# EUROPEAN PATENT SPECIFICATION

(11) **EP 3 664 770 B1**
(45) Date of publication and mention of the grant of the patent: **04.12.2024**
(21) Application number: 18843106.8
(22) Date of filing: 08.08.2018
(51) Int. Cl.: A61K 8/00

(54) **THERAPEUTIC GAS INFUSED COSMETIC AND METHODS OF INFUSION**
THERAPEUTISCHES GASINFUNDIERTES KOSMETIKUM UND INFUSIONSVERFAHREN
PRODUIT COSMÉTIQUE DANS LEQUEL UN GAZ THÉRAPEUTIQUE EST PERFUSÉ, ET PROCÉDÉS DE PERFUSION

(30) Priority: 08.08.2017 US 201762542726 P
(43) Date of publication of application: 17.06.2020
(73) Proprietor: Tetrous, Inc., Sherman Oaks, CA 91403 (US)
(72) Inventor: PATT, Bradley E., Northridge, CA 91324 (US); CARTER, Andrew J., Stow, MA 01775 (US); HUSSAIN, Ali, Northridge, CA 91324 (US)
(74) Representative: Hutter, Anton
(86) International application number: PCT/US2018/045882
(87) International publication number: WO 2019/032756

(56) References cited:
- WO-A1-2016/166347
- WO-A1-2016/166347
- WO-A1-2017/128523
- US-A1- 2006 193 878
- US-A1- 2006 193 878
- US-A1- 2006 244 160
- US-A1- 2012 004 598
- US-A1- 2012 004 598
- US-B1- 9 527 046
- US-B2- 7 703 483

## Description

### CROSS-REFERENCE TO RELATED APPLICATION(S)

The present application claims priority to and the benefit of U.S. Provisional Application Serial No. 62/542,726 entitled "Therapeutic Gas Infused Cosmetic and Methods of Infusion," filed on August 8, 2017.

### BACKGROUND

Skin cells rely on the constant supply of therapeutic gases to generate energy to drive the numerous cellular functions for maintaining healthy skin, and healthy looking skin. The processes that demand therapeutic gases (e.g., oxygen) include extracellular matrix collagen production, elastin proliferation, general cell metabolism amongst others, all of which are necessary for sustaining healthy, toned, and elastic non-aged appearing skin. The concentration of therapeutic gases in skin is reduced with age due to compromised vasculature as a result of aging, pollution, sun exposure, smoking, alcohol use, or health related impediments, the risk for which increases with age. See for example US2012/004598.

### SUMMARY

The invention is as defined by the claims, and any other aspects, configurations, or embodiments set forth herein not falling within the scope of the claims are for information only. Additionally, any references in the description to methods of treatment refer to the compounds, pharmaceutical compositions, and medicaments of the present invention for use in a method of treatment of the human (or animal) body by therapy (of for diagnosis).

In some embodiments of the present disclosure, a first system for infusing oxygen into a composition for treating skin, the system including a source of oxygen, a container for holding the composition, and a means of fluid communication with the source of oxygen for delivering the oxygen. The means for fluid communication is a cannula, the cannula capable of being inserted into the container. The cannula may have a plurality of openings along its length.

The first system may comprise a valve for relieving pressure. The source of gas in the system is oxygen. The source of gas in the system may be in a cylinder or a canister. The source of gas of the system may be or may be in an oxygen concentrator.

In some embodiments of the present invention, the means for fluid communication of the first system may be disposed at the bottom or the top of the container. In some embodiments, the system includes a switch disposed between the container and the source of gas, the switch being capable of allowing an influx of gas into the container upon actuation of the switch by contact of the switch with the container. The system may also include a pump positioned in the container where the pump is positioned in the container and is capable of pumping the composition out of the container.

In some embodiments of the present disclosure the means for fluid communication in the first system is a needle or a cannula. In some embodiments, the cannula has a plurality of openings along its length.

In some embodiments of the present disclosure, a second system for infusing a gas into a composition for treating skin includes a source of gas, a first container for holding the bulk composition, a second container that holds an amount of the composition for a single application in which oxygen infusion occurs, a means for transferring an amount of the composition for a single application from the bulk container into the infusion container, a means for fluid communication with the source of gas for delivering the gas to the infusion chamber, and a means for dispensing a single dose of the composition from the infusion container. In some embodiments, the means of fluid communication of the second system is a cannula, where the cannula is capable of being inserted into the infusion chamber. In some embodiments, the cannula has a plurality of openings along its length. The second system may also include a valve for relieving pressure. In some embodiments, the source of gas of the second system is oxygen. The source of gas of the system is in a cylinder or a canister. The source of gas of the second system may be an oxygen concentrator. The means of fluid communication of the second system may be disposed at the bottom or the top of the container. The second system may also include a switch disposed between the container and the source of gas, the switch being capable of allowing an influx of gas into the container upon actuation of the switch by contact of the switch with the container.

The second system according to embodiments of the present invention includes a pump positioned in the container, the pump being capable of pumping the composition out of the bulk container into the infusion container. The pump of the second system may be positioned in the container and the pump may be capable of pumping the composition out of the infusion container and dispensing it. The means of communication of the second system may be a needle. In some embodiments, the cannula of the second system has a plurality of openings along its length. In some embodiments, the means for fluid communication of the second system is a tube.

A method of making a gas-infused cosmetic composition, the method including placing a cosmetic composition in a container having a cannula, and actuating a flow of gas from the source of gas through the cannula into the cosmetic composition to form a gas-infused cosmetic composition. The cosmetic composition may include a fluorocarbon. The perfluorocarbon may be, for example, a perfluorocarbon. Examples of perfluorocarbon include perfluorodecalin, perfluorohexane, perfluoroperhydrophenanthrene, perfluorobutylamine (PFTBA or PFTBM), perfluorooctylbromide (PFOB), perfluoro-n-octane, octafluoropropane, perfluorodichlorooctane, perfluorodecalin (PFD), perfluorotripropylamine, perfluorotrimethylcyclohexane, perfluoromethyladamantane, perfluorodimethyladamantane, perfluoromethyldecaline, perfluorofluorene, diphenyldimethylsiloxane, hydrogen-rich monohydroperfluorooctane, alumina-treated perfluorooctane, or mixtures thereof.

**The** method of the present disclosure includes the flow of gas from the source of gas through the means of fluid communication into the composition at a pressure of 100 to 900 mbar. The method of the present disclosure may also include a gas-infused cosmetic composition that is saturated or supersaturated with a gas or a mixture of gases. The gas-infused cosmetic composition may be saturated or supersaturated with oxygen. The gas-infused cosmetic composition is held at a pressure above atmospheric pressure.

In some embodiments of the present disclosure a method for treating skin of a subject includes applying the gas-infused cosmetic composition according to embodiments of the present disclosure and made by any of the systems of the present disclosure, where the method includes applying the composition to the skin of a subject within a second or up to 8 hours after making the gas-infused cosmetic composition.

In some embodiments of the present disclosure, a gas-infused cosmetic composition is saturated or supersaturated with oxygen. In some embodiments, this saturated or supersaturated composition forms a mousse or foam when dispensed from the container of the first system or the second system of the present disclosure.

### BRIEF DESCRIPTION OF THE DRAWINGS

Figure 1 is a schematic of the apparatus for oxygenation of a composition, according to some embodiments of the present invention.
Figure 2 is a schematic of the apparatus for oxygenation of a composition, according to some embodiments of the present invention.
Figure 3 is a schematic of the apparatus for oxygenation of a composition, according to some embodiments of the present invention.
Figure 4 is a schematic of the apparatus for oxygenation of a composition, according to some embodiments of the present invention.
Figure 5 is a schematic of the apparatus for oxygenation of a composition, according to some embodiments of the present invention.
Figure 6 is a schematic of the apparatus for oxygenation of a composition, according to some embodiments of the present invention.
Figure 7 is a schematic of the apparatus for oxygenation of a composition, according to some embodiments of the present invention.

### DETAILED DESCRIPTION

The invention is as defined by the claims and any other aspects, configurations or embodiments set forth herein not falling within the scope of the claims are for information only. Additionally, any references in the description to methods of treatment refer to the compounds, pharmaceutical compositions, and medicaments of the present invention for use in a method of treatment of the human (or animal) body by therapy (of for diagnosis).

Aspects of embodiments of the present disclosure are directed to systems and methods for infusing therapeutics gases into a cosmetic composition for delivery of the gas-inflused composition to the skin of a subject.

There are a number of mechanisms to increase oxygen delivery to skin cells. Fluorocarbons (FCs) such as perfluorocarbons (PFCs) may be used as stable, non-toxic, water-immiscible gas-transporting chemicals. Thus FCs have found many utilities in biomedical applications. They are able to dissolve gases such as oxygen, nitrogen, and carbon dioxide in quantities far in excess of other materials such as water and hydrocarbon materials. FCs can dissolve and thus transport enough oxygen (e.g., from about 20% up to 100% oxygen) to be considered as possible blood substitutes.

It has been disclosed that the superficial surface of the skin, (e.g., up to 0.5 mm in depth) absorbs oxygen not only through vascular blood supply but also from the oxygen in the air. It has also been disclosed that dissolved oxygen will penetrate skin more effectively than gaseous oxygen (Roe, D.F., Gibbins, B.L. and Ladizinsky, D.A., 2010. Topical dissolved oxygen penetrates skin: model and method. Journal of Surgical Research, 159(1), pp.e29-e36.).

In addition, topical oxygen is more effective than vascular oxygen supply for superficial skin. Accordingly, FCs with dissolved oxygen are an effective way of supplying dissolved oxygen topically to the superficial surface of the skin.

FC emulsions may be used to deliver oxygen to wounded or damaged skin. FC compositions may be processed for wound treatment modalities. FCs have been shown to have advantageous effects on skin tone, aged skin, and environmentally damaged skin.

Fluorocarbons are hydrophobic in nature and immiscible with water based and organic based systems. Hence, fluorocarbons may be formulated in emulsions, dispersions, or gels. The stability of these emulsions is a major challenge in their utility. Many strategies are used to increase the stability of fluorocarbon emulsions such as the use of stabilizing agents, decreasing fluorocarbon droplet sizes and the development of effective surfactants. In addition, many ingredients are used in cosmetic formulations, and some of these ingredients are susceptible to oxidation. Thus, it is difficult to develop stable products utilizing such ingredients when incorporating high levels of oxygenation when the products are intended to have substantial shelf life.

Packaging for gas infused cosmetics intended to preserve a higher concentration of a specific gas in the cosmetic than the concentration of that gas in the ambient air is costly. Moreover, specific packaging such as air-tight packaging and gas-barrier packaging or hermetic sealing capability that are required to maintain higher gas concentrations in the cosmetic within the package than outside the package are costly and difficult to implement adding further cost. The specialized materials that are required to act as a barrier include polymers such as ethylene vinyl alcohol and metals such as aluminum (Al). Multi-layered materials are generally required. The cost of the cosmetic is an important factor.

An apparatus has been developed that may be used to improve the effectiveness of the cosmetic composition by increasing the dissolved gases in the cosmetic composition just prior to use or application of the cosmetic composition rather than at the time of manufacture of the cosmetic composition. As used herein, "just prior" and "prior to" refers to the time elapsing between gas infusion into the composition and application of the composition, where the time includes no time between the gas infusion step and the application and up to about 8 hours between gas infusion into the composition and application of the composition. In some embodiments of the present invention, the time between the gas infusion step and the application is about 1 second up to about 8 hours. In some embodiments, the time between the gas infusion step and the application is about 30 seconds up to about 8 hours. In some embodiments, the time between the gas infusion step and the application is about 1 minute up to about 8 hours. In some embodiments, the time between the gas infusion step and the application is about 2 minutes up to about 8 hours. In some embodiments, the time between the gas infusion step and the application is about 10 minutes up to about 8 hours. In some embodiments, the time between the gas infusion step and the application is about 15 minutes up to about 8 hours. In some embodiments, the time between the gas infusion step and the application is about 20 minutes up to about 8 hours. In some embodiments, the time between the gas infusion step and the application is about 30 minutes up to about 8 hours. In some embodiments, the time between the gas infusion step and the application is about 1 hour up to about 8 hours. In some embodiments, the time between the gas infusion step and the application is about 90 minutes up to about 8 hours. In some embodiments, the time between the gas infusion step and the application is about 2 hours up to about 8 hours. In some embodiments, the time between the gas infusion step and the application is about 3 hours up to about 8 hours. In some embodiments, the time between the gas infusion step and the application is about 4 hours up to about 8 hours. In some embodiments, the time between the gas infusion step and the application is about 5 hours up to about 8 hours. In some embodiments, the time between the gas infusion step and the application is about 6 hours up to about 8 hours. In some embodiments, the time between the gas infusion step and the application is about 7 hours up to about 8 hours.

Infusing gas into the cosmetic composition prior to use obviates the need for dissolving high concentrations of gas in the cosmetic during manufacture of the composition, thus improving the stability of the cosmetic formulations and eliminating shelf-life concerns of the cosmetic composition product and packaging for improved quality of the composition.

Some embodiments of the present invention include a cosmetic composition that is suitable for infusion with a therapeutic gas and methods for gas infusion. The use of cosmetic compositions that include perfluorocarbons provide a means of incorporating substantial quantities of a therapeutic gas into a cosmetic composition. The use of emulsified formulations provides materials that are pleasing to the skin and may be spread over, and/or absorbed into the skin.

A number of different therapeutic gases may be used for infusion into a cosmetic composition. Examples of therapeutic gases for cosmetic compositions include oxygen, carbon dioxide, ozone, nitrogen, hydrogen sulfide, nitric oxide, xenon, hydrogen, or suitable mixtures thereof.

In one embodiment oxygenation or infusion of the formulation with oxygen in close proximity in time to, or at the time of use minimizes the risk of oxidative damage to the formulation ingredients during storage prior to use, and the need for oxygen barrier packaging.

An embodiment of the present invention also includes a cosmetic dispensing methodology to impregnate the cosmetic formulation with gases, which involves: a) a gas cylinder connected to the cosmetic containing container, b) an optional microbubbling needle used for gas impregnation, and c) a pressure relief valve for controlling the pressure in the container. This gas impregnation being done at the time of use.

In an embodiment of the present invention the gas is introduced directly into the container with no microbubbling needle or pressure relief valve. In this instance the cosmetic will be held at pressures above atmospheric pressure and will have a significant greater quantity of gas dissolved in it than would be possible at atmospheric pressure. Upon release from the container the excess gas forms bubbles creating a mousse, or foamed, formulation. This approach may be particularly favored for use as an "oxygen face mask" for example, or to "kick start" a healing process. Other methods of infusing the gas in close proximity in time to the time of use of the cosmetic will be readily apparent to those of ordinary skill in the art.

The cosmetic composition and gas impregnation process as disclosed herein provides a cosmetic product and production modality that may be dispensed at salons, consumer sites, healthcare offices, in the home, or carried by a user as a mobile dispensing unit to improve oxygenation of the skin, which will minimize wrinkles, benefit skin tone, promote skin color, radiance, and youthful skin appearance amongst other beneficial uses.

The cosmetic composition may be provided in large bulk containers at the time of use. The dose of material suitable for use e.g. 5-50 ml for a total body massage or 0.5-5.0 ml for a facial treatment can be dispensed into a simple pump container. This container is then placed into an apparatus wherein a needle with micro perforations along its length is placed into the cosmetic formulation. The container is sealed against the apparatus and gas is introduced into the cosmetic composition. This gas may be oxygen, as well as other gases such as carbon dioxide, ozone, nitrogen, hydrogen sulfide, nitric oxide, xenon and hydrogen, or mixtures thereof.

Perfluorocarbons have very high inherent gas solubility. Cosmetic compositions including perfluorocarbons may be used as delivery vehicles for a variety of gases including, but not limited to oxygen.

The gas impregnated fluorocarbon emulsions described according to embodiments of the present invention may be applicable for the treatment of wounds, burns, scrapes, bruises, scratches, and other skin impediments. The local delivery of gas via the emulsions can also be incorporated into pharmaceuticals that include antioxidants, moisturizing agents, therapeutic factors, nutritional elements, anti-microbial, anti-biotic, anti-fungal, and other healing agents.

The term "skin" as used in this invention indicates any skin tissue on the entire body including the scalp.

The terms cosmetic, cosmetic composition, cosmetic formulation, cosmetic cream, cream, formulation and composition are used interchangeably and denote a material designed to be applied to the skin, scalp, hair follicles, or hair. As used herein, in addition to creams and ointments, the cosmetic composition may also include a washing fluid or washing solution having water as its most abundant component.

While the delivery of oxygen is an embodiment of this invention, other gases or mixtures of gases with therapeutic benefit may be added to or substituted for oxygen.

In some embodiments, the cosmetic formulations of the present invention include an oxygen /gas carrier that is a material with high inherent gas solubility.

In some embodiments of the present invention, the oxygen / gas carrier is a perfluorocarbon (PFC). These materials have a very high inherent solubility for oxygen and other gases. Non-limiting examples of PFCs include perfluorodecalin, perfluorohexane, perfluoroperhydrophenanthrene, perfluorobutylamine (PFTBA or PFTBM), perfluorooctylbromide (PFOB), perfluoro-n-octane, octafluoropropane, perfluorodichlorooctane, perfluorodecalin (PFD), perfluorotripropylamine, perfluorotrimethylcyclohexane, perfluoromethyladamantane, perfluorodimethyladamantane, perfluoromethyldecaline, perfluorofluorene, diphenyldimethylsiloxane, hydrogen-rich monohydroperfluorooctane, alumina-treated perfluorooctane, mixtures thereof, or any suitable oxygen carrier.

In order to make a cosmetic composition that is pleasing in its ability to spread onto, and be absorbed into, the skin, FCs which are inherently very hydrophobic, may be incorporated into an emulsion by use of an emulsifying agent.

The emulsifying agent may be a non-fluorinated chemical or compound such as hydrogenated phospholipids including, but not limited to, hydrogenated phosphatidylcholine, phosphatidylserine, phosphatidylinositol, phosphanolipids, phosphatidic acid, and other mixtures thereof.

In some embodiments, the cosmetic composition includes from about 0.1% to about 90% fluorocarbons by weight.

It is noted that under ambient conditions when exposed to air, FCs will contain the same ratio of gases found in air.

Saturated or supersaturated forms of the perfluorocarbon may be made by exposing the FC to a gas at pressure above ambient under temperature and time conditions necessary to displace other gases in the FC with the desired gas. For example, the FC may be saturated or supersaturated by exposing the FC to oxygen. In some embodiments, the oxygen may be in the form of molecular oxygen, at pressures at or above ambient and under temperature and time conditions necessary to displace the other gases. The supersaturation of the FC may be undertaken at the time of manufacture in which case the cosmetic may be packaged in a gas barrier package. The gas barrier package may include multiple layers designed to modulate the diffusion of gases across the barrier. The barrier layers may be chosen to accelerate certain gas diffusions in one direction across the layer or the other. The FC may be supersaturated in proximity to the time of, or at the time of application. A preferred method of effecting the saturation is to bubble gas through the formulation using a needle with micro-perforations in it in close proximity (from about one second up to about 8 hours) in time to use. The use of multiple small perforations will lead to the production of numerous very small bubbles that will more effectively introduce the new gas.

The introduction of the gas may assist in mixing the ingredients in the cosmetic prior to use. In some embodiments, additional means of mixing of the cosmetic ingredients may be carried out by any suitable means. For example, mixing of the cosmetic formulation may be carried using any mixing aid such as a magnetic stir bar, by vortex, an agitator, or a blender.

In some embodiments, the system may optionally include apressure relief valve to allow venting of the unwanted gas and prevent or reduce overpressurisation.

Cosmetics in which the formulation has been oxygenated will be unstable and will seek to return to the proportion of gases found in normal air unless the material is kept in barrier packaging. This barrier packaging must maintain an oxygen barrier. Such packaging materials exist but are expensive. It is thus beneficial to infuse gas into the formulation in close proximity (one second to several hours) in time prior to use so that conventional packaging materials can be used

The long term stability of components and cosmetic formulations that are susceptible to oxidation is compromised if the cosmetic composition is oxygenated. Thus it can be beneficial to oxygenate these types of compositions at or near the time of use, thus avoiding the need to maintain stability over long term shelf storage.

For cosmetic compositions that contain components that are particularly susceptible to oxidative damage the compositions may be infused with nitrogen or some other inert gas at the time of manufacture and stored in gas barrier packaging. Infusion with a therapeutic gas such as oxygen that supplants some or all of the temporary gas may then be done in close proximity (one second to several hours) in time prior to use.

Perfluorocarbon materials may be formed into emulsions by, for example, vortexing a dispersing agent solution with a FC. The emulsions may be thickened by the addition of a water soluble polymer into the water phase of the emulsion. Alternatively, the emulsion can be concentrated by use of a centrifuge, or by any suitable method known in the art.

Non-limiting examples of dispersing agents include glycerols, phospholipids, lecithins, surfactants, and/or polyoxamers.

Cosmetic compositions may include a number of other ingredients designed to provide specific benefits to the user's skin in terms of e.g. moisturization, reduction of irritation, anti-aging molecules, as well as components to improve the texture, feel, and/or scent of the cosmetic.

Additives to the cosmetic formulation include emollients, anti-oxidants, essential oils, such as aloe vera, collagen, glyceryl stearate, capric triglyceride, stearic acid, shea butter, cetyl alcohol, pentylene glycol, propanediol, tranexamic acid, camellia oleifera, myristyl myristate, phenoxyethanol, cyclopentadecanolide, hydroxyethyl acrylate, hyaluronic acid, gellan, carbomer, and alginate.

In some embodiments of the present invention, the impregnation of gas into the cosmetic composition occurs in close proximity (one second to several hours) in time prior to the intended use of the cosmetic composition. For example, gas infusion into the cosmetic composition may occur at the spa, dermatologist's office, home, healthcare center, or in a portable device.

The impregnation of oxygen may be performed until the fluorocarbon reached its saturation point with oxygen. For example, the oxygen gas with a pressure of 100 to 900 mbar (about 1.45 pressure per square inch (psi) to about 13.1 psi) is bubbled in the formulation at ambient temperature (16 to 30 °C) for a time period of about 10 seconds up to about 20 minutes. In some embodiments, the oxygen gas is provided to the cosmetic composition at a pressure of about 200 to about 900 mbar. In some embodiments, the oxygen gas is provided to the cosmetic composition at a pressure of about 300 to about 900 mbar. In some embodiments, the oxygen gas is provided to the cosmetic composition at a pressure of about 400 to about 900 mbar. In some embodiments, the oxygen gas is provided to the cosmetic composition at a pressure of about 500 to about 900 mbar. In some embodiments, the oxygen gas is provided to the cosmetic composition at a pressure of about 600 to about 900 mbar. In some embodiments, the oxygen gas is provided to the cosmetic composition at a pressure of about 700 to about 900 mbar. In some embodiments, the oxygen gas is provided to the cosmetic composition at a pressure of about 800 to about 900 mbar.

While it is anticipated that the most common gas of choice for saturation of the formulations will be oxygen it is also possible that other gases may be used, or may be mixed with the oxygen. For example, hydrogen has antioxidant characteristics and may also be incorporated into the formulations.

While the cosmetic composition according to embodiments of the present invention may be oxygenated during manufacture, it is preferred that it is not. That is, cosmetic formulations which are particularly susceptible to oxidation they may be impregnated with nitrogen at the time of manufacture to enhance the stability. In this instance the product may be infused with nitrogen and then appropriately packaged with a septum in the base of the container to allow for further gas infusion, for example, oxygenation.

The infusion units may be designed to accommodate a large gas cylinder for use at salons / spas, dermatologist offices, or may have a smaller gas cylinder or cartridges for a home use device or even smaller gas cylinder for a portable device.

Oxygen may be used, but in some instances it may be desired to utilize a mix of gases, so that for example a mixture of oxygen and hydrogen are used. Suitable gases for infusion into the cosmetic composition include oxygen, hydrogen, carbon dioxide, ozone, nitrogen, hydrogen sulfide, nitric oxide, xenon, or mixtures thereof.

In some embodiments of the present invention, components of the gas saturation device include a micro bubbling cannula or needle and a source or sources of gas.

The micro bubbling cannula or needle allows for communication between a gas source (e.g. a gas cylinder) and the cosmetic composition. The cannula may be inserted into a container that houses the cosmetic compositions. In some embodiments, the cannula is a needle. In some embodiments, the needle is perforated, for example, by laser cutting. The small perforations in the length of the needle allow for a pressurized gas to form micro bubbles in the formulation enhancing the rate of gas transfer. The valve, or switch that regulates flow of gas from the gas source and the cosmetic composition may also act to modulate the gas pressure to influence gas dispersion in the cosmetic composition meaning that in some cases the needle is not required.

A source of gas may be in any suitable form. For example, the source of gas may be a gas cylinder or canister. For use in spas, dermatologist offices etc. a larger cylinder may be used. For personal use a smaller cylinder (e.g., canister) and in some designs a "micro bullet" cylinder suitable for single use may be provided.

The source of gas may also be an oxygen concentrator that uses a filter to remove nitrogen from compressed air, thus increasing the oxygen concentration. Such devices are regularly used for individuals who need help with their breathing.

In some embodiments of the present invention, insertion of the gas cylinder activates the gas infusion process. In some embodiments, an apparatus designed for multiple uses includes a container housing the cosmetic composition and the container sealed against the apparatus with a vent and switch that will activate gas flow into the cosmetic composition. The seal retains the cosmetic composition in the container while the vent will allow the unwanted gases to escape.

In some embodiments of the present invention, the oxygen and/or other gases in the cosmetic composition is at a pressure above atmospheric pressure and upon dispensing from the pressurized container the dissolved gas forms bubbles such that the composition becomes a mousse or foam. The mousse has a high proportion of gas that is immediately available to the skin. The oxygen and/or other gases are bubbled into the cosmetic composition upon application to the skin creating a pleasing effect.

It is possible to oxygenate, or gas saturate a number of different cosmetic formulations that may be utilized for different purposes. So for example, a washing water having a high proportion of water could be treated for use as an oxygenated cleanser, for skin or hair. A face cream with a high proportion of perfluorocarbon materials when oxygenated will have a very high oxygen content due to the very high oxygen solubility in perfluorocarbons and will have great utility as a face cream to reduce the appearance of wrinkles and to bulk or plump the skin.

In some embodiments of the present invention, the infusion apparatus design includes a user interface that controls the gas valve to regulate the concentration of the gas and/or ratio of gases (e.g., oxygen, nitrogen, carbon dioxide, hydrogen) used to impregnate the emulsion.

In some embodiments of the present invention, insertion of the gas cylinder to the container activates the infusion process. In some aspects of this embodiment, the container is a one-time use, and therefore is made inexpensively such that after attachment and activation with the gas cylinder and use of the infused product, the container may be disposed.

In some designs of the apparatus according to embodiments of the present invention, the predetermined gas ratio is formulated into the gas cylinder. Thus a gas cylinder may contain 100% oxygen or may contain between one and 30% hydrogen, and/or other desired gases.

In some designs of the apparatus according to embodiments of the present invention, the gas cylinder contains 90% oxygen and 10% nitrogen, and/or other desired gases.

**As** used herein, the term "means of communication," "means of fluid communication," and "fluid communication," are used interchangeably to refer to a conduit or channel for transporting a fluid, such as a gas. Non-limiting examples of the means of communication include a cannula, a channel, a tube, or a needle.

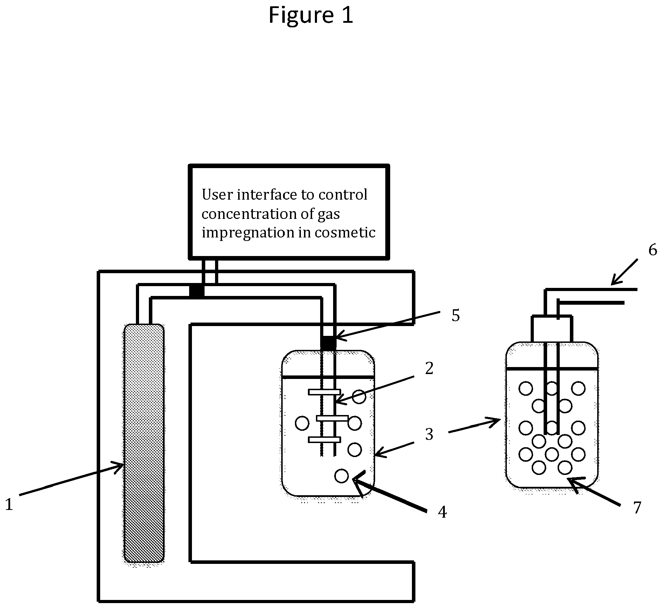

In some embodiments of the present invention, the apparatus for oxygenation of a cosmetic as shown in Figure 1 comprises a gas cylinder (1), and a microbubbling needle (2) that has a tip and penetrating holes (i.e., openings) along its length. The cosmetic composition to be gas-infused (4) is placed into an infusion container (3). In salons, spas, other facilities where multiple people are to be treated, as well as at home or as a portable device, wherein multiple gas impregnations can be achieved over time, the cosmetic composition may be provided in bulk in a suitable container (not shown). This bulk cosmetic container will facilitate more than one consumer application of the cosmetic composition with the individual "dose" for a single oxygenation being dispensed into the infusion container (3) proximally in time to its use. The gas infusion into the cosmetic composition may be performed within a few second of use up to several hours prior to use. If required other individual additives such as essential oils can be added to the cosmetic composition prior to oxygenation-the act of oxygenation may also serve to mix the additives into the composition. The infusion container is placed into the apparatus such that the needle is in the cosmetic composition and the container is raised up so that the top of the container contacts the switch (5). This switch seals the container and also activates a valve to allow gas to be bubbled into the cosmetic composition. The switch seal is vented to prevent excessive pressure build up. The container is removed from the apparatus and a dispensing pump top (6) is put onto the container (3). The treated cosmetic composition (7) is now available for use.

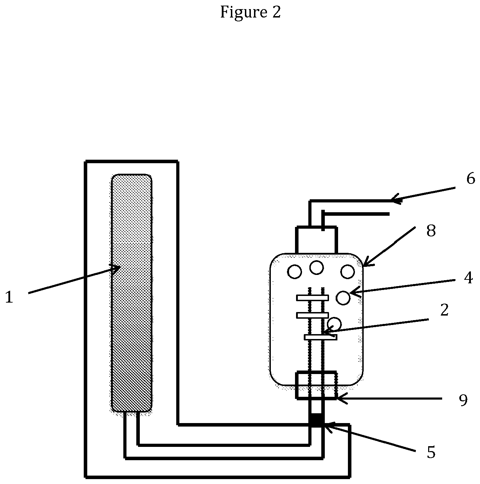

In some embodiments of the present invention, the apparatus shown in Figure 2 includes the cosmetic composition in a prefilled container (8) that has a dispensing pump (6) already in place. In this variation of the apparatus, the microbubbling needle (2) is introduced into the container through a self-sealing septum (9). When the container contacts the vented switch (5) gas flow from the gas cylinder (1) into the cosmetic composition (4) is activated.

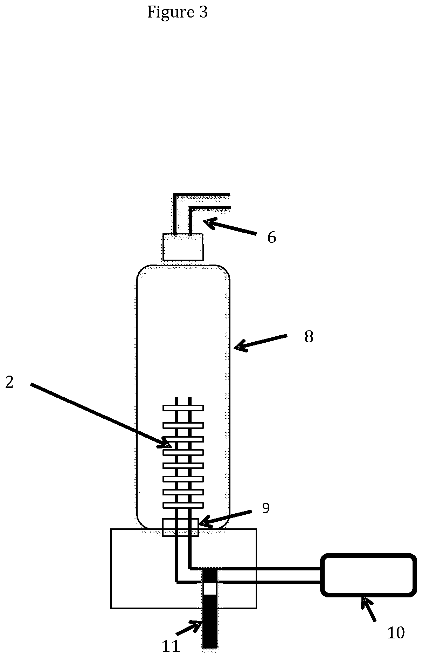

In some embodiments of the present invention, the apparatus shown in Figure 3 includes the cosmetic composition in a prefilled container (8) that can be attached to a perforated needle (2) through a self sealing septum (9) in the base of the container that allows gas in-flow from a customized gas cartridge (10) by pushing an activating valve button (11). After oxygenation the container (8) can be removed from the base unit. The cosmetic composition is dispensed through the dispensing pump top (6)

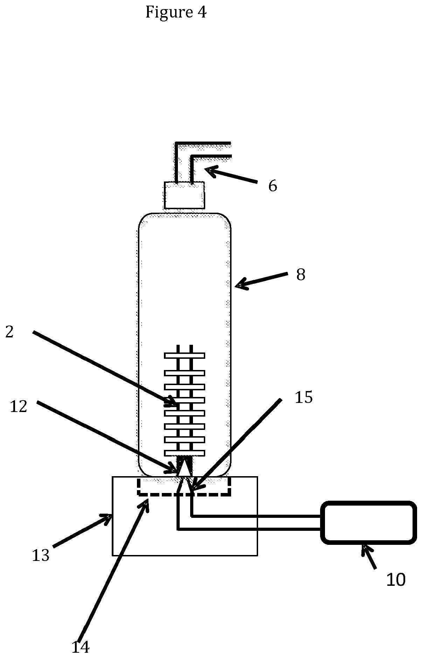

In some embodiments of the present invention, the apparatus shown in Figure 4 includes the cosmetic (emulsion) composition in a prefilled container (8) with a perforated needle (2) embedded in the container. For example, the proximal end of the needle may be affixed to a female gas valve or gas micro-valve connector (12) that is sealed hermetically against the container (8). The container may be seated permanently or intermittently on a base unit (13) that may have, for example, an indentation (14) with a spring feature to provide resistance against insertion of the container (8) and guide rails. The holding fixture has a mating male micro connector or valve (15) (such as a bayonet type of connector) that allows gas inflow into the prefilled container (8) from a customized gas cartridge (10) by pressing the container (8) forcibly into the fixture (14) which action opens the valve (12). The cosmetic is dispensed through the dispensing pump top (6). In a further variant of the apparatus shown in Figure 4 the act of applying pressure against the valve on the gas cylinder causes activation of the gas flow.

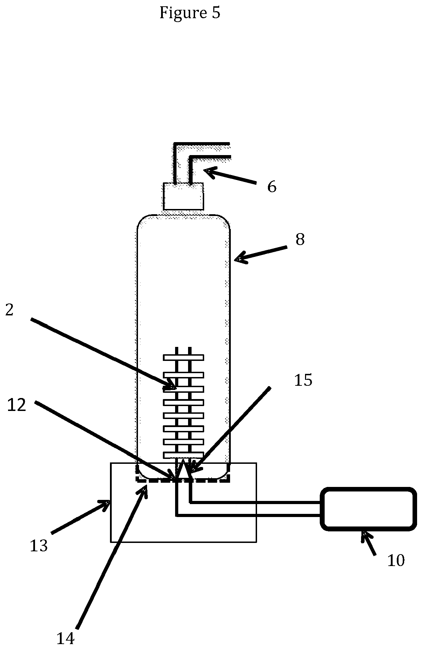

In Figure 5 the apparatus shown in Figure 4 is shown in the activated state wherein the act of insertion of the gas cylinder causes activation of the gas flow.

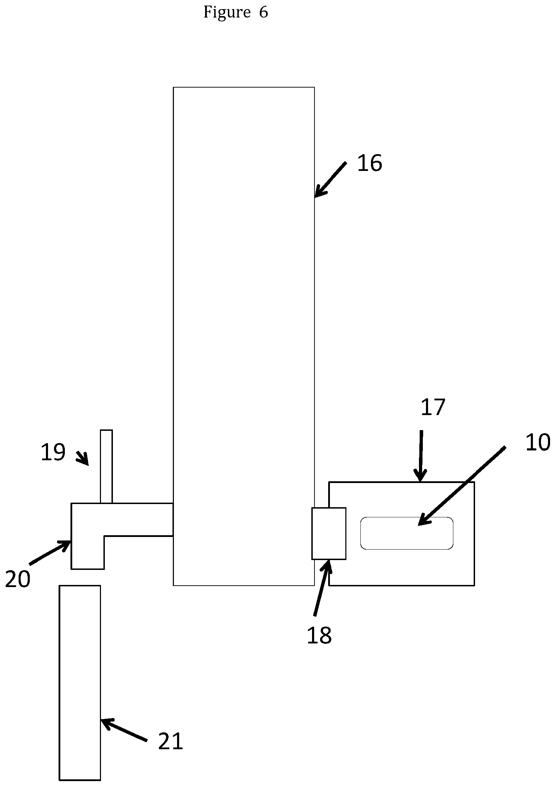

In Figure 6 the cosmetic formulation is placed in a container (16). A gas cartridge (10) is placed in the holder (17) (e.g., a threaded holder). Turning the holder causes the cartridge to be open (e.g., by puncture) to a means of fluid communication (18) (e.g., a valve or channel) to thereby allow the gas to enter the container (16). It should be noted that in this embodiment it is not necessary to use a perforated needle to effect mixing of the oxygen into the cream as the oxygen in the cartridge (10) is at a much higher pressure than the pressure in the container (16) and the turbulence effected by the exit of the gas into the container is sufficient to cause the mixing. A switch (19) allows the pressurized cosmetic to flow through the outlet (20) where a portion of the cosmetic can be filled into a smaller container (21) for use. Such an apparatus could be particularly useful for use in a treatment center where one container of oxygenated product would be sufficient for use for the day (e.g., up to about 8 hours). Alternatively the cream could be dispensed directly onto a hand for application to the user. Upon dispensing the cream from the container the pressurized cream will be subject to ambient pressure and thus the oxygen that is infused in the cream will bubble causing the cream to form a lower density mousse wherein the oxygenated cream contains bubbles of oxygen.

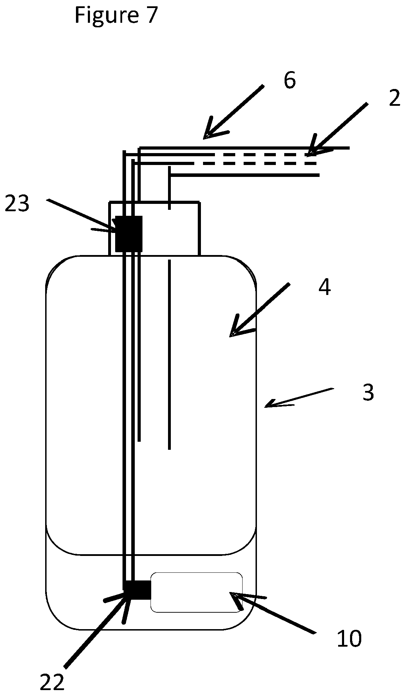

In Figure 7 another embodiment intended to provide an oxygenated cream or mousse of cosmetic cream is shown. The cosmetic cream (4) is supplied in a container (3) that has a small oxygen cylinder (10) fitted into its base. The connector (22) allows attachment of the gas cartridge and also acts as a regulator to regulate pressure. An actuator (23) activates gas flow when the plunger (6) is depressed forcing oxygen through the perforated needle (2) into the cosmetic cream as it is being dispensed. Note that the end of the perforated needle (24) is not open so that the oxygen is forced into the cream. The back flow valve (25) ensures that oxygen does not go back into the cream (4) in the container (3). While in the outflow tube of the dispenser (6) the oxygen in the cream will be above atmospheric pressure so that upon exit, as the pressure normalizes to atmospheric pressure the cream will form a mousse.

The foregoing are non-limiting examples according to some embodiments of the present invention.

The following Examples are presented for illustrative purposes only, and do not limit the scope or content of the present application.

### EXAMPLES

**Example 1.** An aqueous component was produced by adding 0.1 g/ml of Pluronic F127, 0.03 g/ml of Aloe Vera, 0.02 g/ml of collagen and 0.005 g/ml of benzyl alcohol as a preservative. An emulsion of this component and perfluorodecalin was produced at a volumetric ratio of 3:2. This material was then thickened further by mixing at a ratio of 5:2 with a 0.02 g/ml high molecular weight hyaluronic acid aqueous solution. The formulation was oxygenated by placing a 18G needle connected to an oxygen cylinder into the cream. The oxygen content of the gas phase of the cream was monitored using a PreSens Microx 4 oxygen sensor placed into the cream. The oxygen content rose from 20% to 100% after a 5-minute infusion. The resultant cream had a pleasant consistency and was easily spread onto the skin and absorbed by the skin.

**Example 2.** An aqueous component was produced by adding 0.1 g/ml of Pluronic F127, and 0.005 g/ml of benzyl alcohol as a preservative. An emulsion of this component and perfluorodecalin was produced at a volumetric ratio of 3:2. This material was then formulated further by mixing at a ratio of 2:1 with a 0.03 g/ml aloe vera, 0.02g/ml high molecular weight hyaluronic acid, 0.06 g/ml collagen, 0.005g/ml benzyl alcohol aqueous solution. The formulation was oxygenated by placing a 18G needle connected to an oxygen cylinder into the cream. The oxygen content of the gas phase of the cream was monitored using a PreSens Microx 4 oxygen sensor placed into the cream. The oxygen content rose from 20% to 100% after a 5-minute infusion. The resultant cream had a pleasant consistency and was easily spread onto the skin and absorbed by the skin.

**Example 3.** An aqueous component was produced by adding 0.1 g/ml of Polysorbate 20. An emulsion of this component and perfluorodecalin was produced at a volumetric ratio of 3:2. This material was then formulated further by mixing at a ratio of 2:5 with a 0.015 g/ml aloe vera, 0.02g/ml high molecular weight hyaluronic acid, 0.03 g/ml collagen, 0.005g/ml benzyl alcohol aqueous solution. The formulation was oxygenated by placing a 18G (gauge) needle connected to an oxygen cylinder into the cream. The oxygen content of the gas phase of the cream was monitored using a PreSens Microx 4 oxygen sensor placed into the cream. The oxygen content rose from 20% to 100% after a 5-minute infusion. The resultant cream had a pleasant consistency and was easily spread onto the skin and absorbed by the skin.

**Example 4.** An aqueous component was produced by adding 0.1 g/ml of Polysorbate 20. An emulsion of this component and perfluorodecalin was produced at a volumetric ratio of 3:2. This material was then formulated further by mixing at a ratio of 2:1 with a 0.015 g/ml aloe vera, 0.02g/ml high molecular weight hyaluronic acid, 0.06 g/ml collagen, 0.005g/ml benzyl alcohol aqueous solution. The formulation was oxygenated by placing a 18G needle connected to an oxygen cylinder into the cream. The oxygen content of the gas phase of the cream was monitored using a PreSens Microx 4 oxygen sensor placed into the cream. The oxygen content rose from 20% to 100% after a 5-minute infusion. The resultant cream had a pleasant consistency and was easily spread onto the skin and absorbed by the skin.

## Claims

1. A system for infusing oxygen into a single application amount of a cream composition to form a mousse composition prior to use for treating skin, the system, comprising:
a source of oxygen(1,10);
a first container for holding a bulk quantity of the cream composition (4);
a second container (3,8) for holding the single application amount of the bulk quantity of the cream composition for oxygen infusion;
wherein the single application amount is 0.5 ml to 50 ml;
a pump capable of pumping the single application amount of the bulk quantity of the cream composition from the first container into the second container;
wherein the source of oxygen comprises a means of communication (2) for delivering the oxygen to the single application amount in the second container at a pressure above atmospheric pressure to form a single application amount of pressurized cream composition; and
a means for dispensing (6) the single application amount of pressurized cream composition out of the second container to ambient pressure, whereby dispensing from the second container forms a single application mousse composition infused with oxygen (7).

2. The system of claim 1, wherein the means of communication (2) with the source of oxygen is a cannula, a tube, a conduit, or a needle.

3. The system of claim 1, wherein the single application amount is 0.5 to 5.0 ml.

4. The system of claim 1, wherein the single application amount is 5.0 ml to 50 ml.

5. The system of claim 1, wherein the pump is disposed at the bottom or the top of the second container.

6. The system of claim 1, further comprising a switch (5) disposed between the second container and the source of oxygen the switch capable of allowing an influx of oxygen into the second container (3,8) upon actuation of the switch by contact of the switch with the second container.

7. A method of infusing oxygen into a single application mousse composition using the system of any one of claims 1-6, the method comprising:
Placing a cream composition (4) in the first container;
pumping the single application amount of the cream composition from the first container into the second container (3,8);
actuating a flow of oxygen from the source of oxygen (1, 10) into the second container at a pressure above atmospheric pressure to form a pressurized cream composition from the transferred amount of the cream composition; and
dispensing the pressurized cream composition out of the second container to ambient pressure to form the single application mousse composition infused with oxygen (7).

8. The method of claim 7, wherein the cream composition comprises an emulsifying agent.

9. The method of claim 8, wherein the emulsifying agent is a hydrogenated phospholipid; preferably the emulsifying agent is hydrogenated phosphatidylcholine, phosphatidylserine, phosphatidylinositol, phosphanolipids, or phosphatidic acid.

10. The method of claim 8, wherein the cream composition comprises a fluorocarbon.

11. The method of claim 10, wherein the fluorocarbon is a perfluorocarbon.

12. The method of claim 11, wherein the perfluorocarbon is selected from perfluorodecalin, perfluorohexane, perfluoroperhydrophenanthrene, perfluorobutylamine (PFTBA or PFTBM), perfluorooctylbromide (PFOB), perfluoro-n-octane, octafluoropropane, perfluorodichlorooctane, perfluorodecalin (PFD), perfluorotripropylamine, perfluorotrimethylcyclohexane, perfluoromethyladamantane, perfluorodimethyladamantane, perfluoromethyldecaline, perfluorofluorene, diphenyldimethylsiloxane, hydrogen-rich monohydroperfluorooctane, alumina-treated perfluorooctane, or mixtures thereof.

13. The method of any one of claims 7-12, wherein the single application mousse composition infused with oxygen is applied to the skin within 8 hours of oxygen infusion.

## Patentansprüche

1. System zum Einbringen von Sauerstoff in eine Einzelanwendungsmenge einer Cremezusammensetzung, um vor der Verwendung zur Behandlung von Haut eine Mousse-Zusammensetzung zu bilden, wobei das System umfasst:
eine Sauerstoffquelle (1,10);
einen ersten Behälter zum Aufnehmen einer Packungsmenge der Cremezusammensetzung (4);
einen zweiten Behälter (3,8) zum Aufnehmen der Einzelanwendungsmenge der Packungsmenge der Cremezusammensetzung zur Sauerstoffinfusion;
wobei die Einzelanwendungsmenge 0,5 ml bis 50 ml beträgt;
eine Pumpe, die in der Lage ist, die Einzelanwendungsmenge der Packungsmenge der Cremezusammensetzung aus dem ersten Behälter in den zweiten Behälter zu pumpen;
wobei die Sauerstoffquelle ein Kommunikationsmittel (2) zum Liefern des Sauerstoffs an die Einzelanwendungsmenge im zweiten Behälter bei einem Druck über dem atmosphärischen Druck umfasst, um eine Einzelanwendungsmenge der unter Druck stehenden Cremezusammensetzung zu bilden; und
ein Mittel zum Abgeben (6) der Einzelanwendungsmenge der unter Druck stehenden Cremezusammensetzung aus dem zweiten Behälter auf Umgebungsdruck, wobei durch Abgeben aus dem zweiten Behälter eine mit Sauerstoff infundierte Mousse-Zusammensetzung für eine Einzelanwendung (7) gebildet wird.

2. System nach Anspruch 1, wobei das Kommunikationsmittel (2) mit der Sauerstoffquelle eine Kanüle, ein Schlauch, ein Röhrchen oder eine Nadel ist.

3. System nach Anspruch 1, wobei die Einzelanwendungsmenge 0,5 bis 5,0 ml beträgt.

4. System nach Anspruch 1, wobei die Einzelanwendungsmenge 5,0 ml bis 50 ml beträgt.

5. System nach Anspruch 1, wobei die Pumpe an der Unterseite oder an der Oberseite des zweiten Behälters angeordnet ist.

6. System nach Anspruch 1, das ferner einen Schalter (5) umfasst, der zwischen dem zweiten Behälter und der Sauerstoffquelle angeordnet und in der Lage ist, bei Betätigung des Schalters durch Kontakt des Schalters mit dem zweiten Behälter einen Sauerstoffeinstrom in den zweiten Behälter (3,8) zu ermöglichen.

7. Verfahren zum Einbringen von Sauerstoff in eine Mousse-Zusammensetzung zur Einzelanwendung unter Verwendung des Systems nach einem der Ansprüche 1-6, wobei das Verfahren umfasst:
Einbringen einer Cremezusammensetzung (4) in den ersten Behälter;
Pumpen der Einzelanwendungsmenge der Cremezusammensetzung aus dem ersten Behälter in den zweiten Behälter (3,8);
Auslösen eines Sauerstoffflusses von der Sauerstoffquelle (1,10) in den zweiten Behälter bei einem Druck über dem atmosphärischen Druck, um aus der übertragenen Menge der Cremezusammensetzung eine unter Druck stehende Cremezusammensetzung zu bilden; und
Ausgeben der unter Druck stehenden Cremezusammensetzung aus dem zweiten Behälter auf Umgebungsdruck, um die mit Sauerstoff infundierte Mousse-Zusammensetzung zur Einzelanwendung (7) zu bilden.

8. Verfahren nach Anspruch 7, wobei die Cremezusammensetzung einen Emulgator umfasst.

9. Verfahren nach Anspruch 8, wobei der Emulgator ein hydriertes Phospholipid ist; vorzugsweise ist der Emulgator hydriertes Phosphatidylcholin, Phosphatidylserin, Phosphatidylinositol, Phospholipide oder Phosphatidsäure.

10. Verfahren nach Anspruch 8, wobei die Cremezusammensetzung einen Fluorkohlenwasserstoff umfasst.

11. Verfahren nach Anspruch 10, wobei der Fluorkohlenwasserstoff ein Perfluorkohlenwasserstoff ist.

12. Verfahren nach Anspruch 11, wobei der Perfluorkohlenstoff ausgewählt ist aus Perfluordecalin, Perfluorhexan, Perfluorperhydrophenanthren, Perfluorbutylamin (PFTBA oder PFTBM), Perfluoroctylbromid (PFOB), Perfluor-n-octan, Octafluorpropan, Perfluordichloroctan, Perfluordecalin (PFD), Perfluortripropylamin, Perfluortrimethylcyclohexan, Perfluormethyladamantan, Perfluordimethyladamantan, Perfluormethyldecalin, Perfluorfluoren, Diphenyldimethylsiloxan, wasserstoffreiches Monohydroperfluoroctan, mit Aluminiumoxid behandeltes Perfluoroctan oder Gemische davon.

13. Verfahren nach einem der Ansprüche 7-12, wobei die mit Sauerstoff infundierte Mousse-Zusammensetzung zur Einzelanwendung innerhalb von 8 Stunden nach der Sauerstoffinfusion auf die Haut aufgetragen wird.

## Revendications

1. Système de perfusion d'oxygène en une quantité d'application unique d'une composition de crème pour former une composition de mousse avant utilisation pour traiter la peau, le système comprenant :
une source d'oxygène (1,10) ;
un premier contenant destiné à contenir une quantité en vrac de la composition de crème (4) ;
un deuxième contenant (3, 8) destiné à contenir la quantité d'application unique de la quantité en vrac de la composition de crème pour perfusion d'oxygène ;
dans lequel la quantité d'application unique est de 0,5 ml à 50 ml ;
une pompe capable de pomper la quantité d'application unique de la quantité en vrac de la composition de crème du premier contenant vers le deuxième contenant ;
dans lequel la source d'oxygène comprend un moyen de communication (2) pour délivrer l'oxygène dans la quantité d'application unique dans le deuxième contenant à une pression supérieure à la pression atmosphérique pour former une quantité d'application unique de composition de crème sous pression ; et
un moyen de distribution (6) de la quantité d'application unique de composition de crème sous pression en dehors du deuxième contenant à la pression ambiante, moyennant quoi la distribution à partir du deuxième contenant forme ainsi une composition de mousse à application unique à perfusion d'oxygène (7).

2. Système selon la revendication 1, dans lequel le moyen de communication (2) avec la source d'oxygène est une canule, un tube, un conduit ou une aiguille.

3. Système selon la revendication 1, dans lequel la quantité d'application unique est de 0,5 à 5,0 ml.

4. Système selon la revendication 1, dans lequel la quantité d'application unique est de 5,0 ml à 50 ml.

5. Système selon la revendication 1, dans lequel la pompe est disposée au fond ou en haut du deuxième contenant.

6. Système selon la revendication 1, comprenant en outre un commutateur (5) disposé entre le deuxième contenant et la source d'oxygène, le commutateur étant capable d'admettre un afflux d'oxygène dans le deuxième contenant (3, 8) lors de l'actionnement du commutateur par contact du commutateur avec le deuxième contenant.

7. Procédé de perfusion d'oxygène dans une composition de mousse à application unique utilisant le système selon l'une quelconque des revendications 1 à 6, le procédé comprenant :
le placement d'une composition de crème (4) dans le premier contenant ;
le pompage de la quantité d'application unique de la composition de crème du premier contenant dans le deuxième contenant (3, 8) ;
l'actionnement d'un flux d'oxygène depuis la source d'oxygène (1, 10) dans le deuxième contenant à une pression supérieure à la pression atmosphérique pour former une composition de crème sous pression à partir de la quantité transférée de la composition de crème ; et
la distribution de la composition de crème sous pression en dehors du deuxième contenant à la pression ambiante pour former la composition de mousse à application unique à perfusion d'oxygène (7).

8. Procédé selon la revendication 7, dans lequel la composition de crème comprend un agent émulsifiant.

9. Procédé selon la revendication 8, dans lequel l'agent émulsifiant est un phospholipide hydrogéné ; de préférence, l'agent émulsifiant est la phosphatidylcholine hydrogénée, la phosphatidylsérine, le phosphatidylinositol, les phosphanolipides ou l'acide phosphatidique.

10. Procédé selon la revendication 8, dans lequel la composition de crème comprend un fluorocarbure.

11. Procédé selon la revendication 10, dans lequel le fluorocarbure est un perfluorocarbure.

12. Procédé selon la revendication 11, dans lequel le perfluorocarbure est choisi parmi : perfluorodécaline, perfluorohexane, perfluoroperhydrophénanthrène, perfluorobutylamine (PFTBA ou PFTBM), perfluorooctylbromure (PFOB), perfluoro-n-octane, octafluoropropane, perfluorodichlorooctane, perfluorodécaline (PFD), perfluorotripropylamine, perfluorotriméthylcyclohexane, perfluorométhyladamantane, perfluorodiméthyladamantane, perfluorométhyldécaline, perfluorofluorène, diphényldiméthylsiloxane, monohydroperfluorooctane enrichi en hydrogène, perfluorooctane traité à l'alumine, ou des mélanges de ceux-ci.

13. Procédé selon l'une quelconque des revendications 7 à 12, dans lequel la composition de mousse à application unique à perfusion d'oxygène est appliquée sur la peau dans les 8 heures suivant la perfusion d'oxygène.
